# EUROPEAN PATENT APPLICATION

(11) **EP 2 896 684 A1**
(43) Date of publication of application: **22.07.2015**
(21) Application number: 13837538.1
(22) Date of filing: 10.09.2013
(51) Int. Cl.: C12M 1/00, C12M 1/32

(54) **MICROWELL PLATE**

(30) Priority: 14.09.2012 JP 2012202238
(71) Applicant: Sumitomo Bakelite Company Limited, Shinagawa-ku Tokyo 140-0002 (JP)
(72) Inventor: TSUKADA Ryouhei, Tokyo 140-0002 (JP); OKUBO Haruo, Tokyo 140-0002 (JP)
(74) Representative: Müller-Boré & Partner Patentanwälte PartG mbB
(86) International application number: PCT/JP2013/074421
(87) International publication number: WO 2014/042162

(57) **Abstract**

The purpose of the present invention is to provide a microwell plate, which is capable of favorably forming cell aggregates and which secures an adequate well volume, and provided is a microwell plate equipped with wells that have openings that open on one surface, wherein: the wells demarcate spaces formed from the openings towards the interior of the microwell plate and the spaces have an opening end portion, a bottom end portion formed from the opening end portion towards the interior of the microwell plate, and a transitional portion (43) for smoothly connecting the opening end portion (41) to the bottom end portion (42); the transverse cross-section of the opening end portion has a polygonal shape; and the bottom end portion is formed so that the transverse cross-sectional area decreases gradually towards the interior.

## Description

### Technical Field

The present invention relates to a microwell plate.

### Technical Background

In recent years, studies on screening of drug efficiency evaluation and differentiation-inducing factors are actively conducted. In such studies, various cell aggregates are used in evaluation. Therefore, a culture method for efficiently forming cell aggregates is desired. Further, in evaluation such as screening, it is necessary that a large number of specimens are quickly processed at once. Therefore, a culture vessel that allows a large number of cell aggregates to be formed and evaluated is desired.

For such a purpose, a microwell plate on which a large number of wells are provided is used. Microwell plates with, for example, 96 wells, 384 wells and 1,536 wells are commercially available and can be used to handle a large number of specimens.

However, the following problems exist for a microwell plate on which a large number of wells are provided. A shape and a size of a microwell plate are standardized (ANSI/SBS-1 2004, ANSI/SBS-2 2004, ANSI/SBS-3 2004, ANSI/SBS-4 2004). Therefore, when the number of the wells is increased, a capacity of each of the wells inevitably decreases. Therefore, for example, in a microwell plate that has more than 1,000 wells as disclosed in

Patent Document 1, the capacity of each well is about a few µL. When performing culture in such low-capacity wells, due to ensuring amounts of nutrition factors and an effect of evaporation of a culture medium, it is very difficult to perform the culture. Further, when drug efficiency evaluation is performed, a reagent in an amount ranging from one half of, to equal to that of a culture solution is added to the wells. However, for low-capacity wells, there is no such room.

In order to avoid such problems and to increase the capacity of the well, it is conceivable to make a longitudinal cross section of the well a quadrangle. However, in such a shape of the well, a bottom surface of the well is a flat surface and thus, it is difficult to form of a uniform cell aggregate.

### Related Art

### Patent Document

Patent Document 1: Japanese Translation of PCT International Application Publication No. 2001-509272.

### Summary of the Invention

### Problems to Be Solved by the Invention

The present invention is accomplished in view of the above problems. A purpose of the present invention is to provide a microwell plate that allows formation of a cell aggregate to be satisfactorily performed and a sufficient well capacity to be ensured.

### Means for Solving the Problems

Such a purpose is achieved by the present invention described in the following aspects (1)-(5).
(1) A microwell plate is provided with wells that each have an opening that opens on a surface on one side. Each of the wells defines a space that is formed from the opening toward an interior of the microwell plate. The space has an opening-side portion, a bottom-side portion that is formed from the opening-side portion toward the interior of the microwell plate, and a transition portion 43 that smoothly connects the opening-side portion 41 and the bottom-side portion 42. The opening-side portion has a cross section that has a polygonal shape. The bottom-side portion is formed in such a manner that an area of a cross section thereof gradually decreases toward the interior.
(2) In the microwell plate described in the aspect (1), a ratio between a depth of the opening-side portion and a combined depth of the bottom-side portion and the transition portion is 3:1 - 4:1.
(3) In the microwell plate described in any one of the aspects (1) and (2), a cross section of the opening-side portion parallel to the opening is in a shape of one of a quadrangle, a pentagon and a hexagon.
(4) In the microwell plate described in any one of the aspects (1) - (3), a cross section of the bottom-side portion parallel to the opening has a circular shape.
(5) In the microwell plate described in any one of the aspects (1) - (4), a number of the wells is 384 or more.

### Effect of the Invention

According to the microwell plate of the present invention, by optimizing the shape of the wells, formation of cell aggregates can be satisfactorily performed and a sufficient well capacity can be ensured.

### Brief Description of Drawings

Fig. 1 illustrates a top view of a microwell plate according to an embodiment of the present invention.
Fig. 2 illustrates an enlarged view of an II portion of Fig. 1.
Fig. 3 illustrates a longitudinal cross-sectional view of a well according to the embodiment of the present invention.

### Mode for Carrying Out the Invention

In the following, a preferred embodiment of a microwell plate of the present invention is described in detail with reference to the drawings.

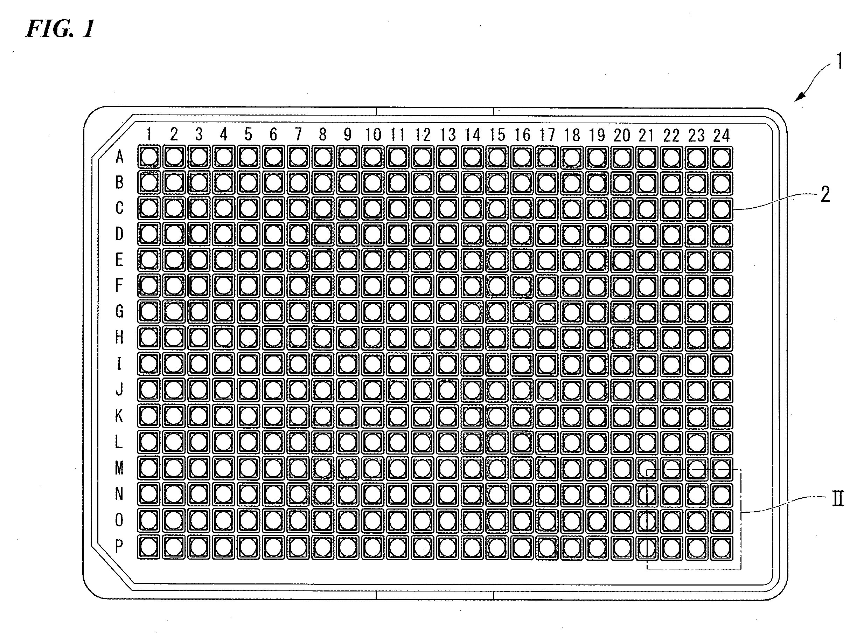

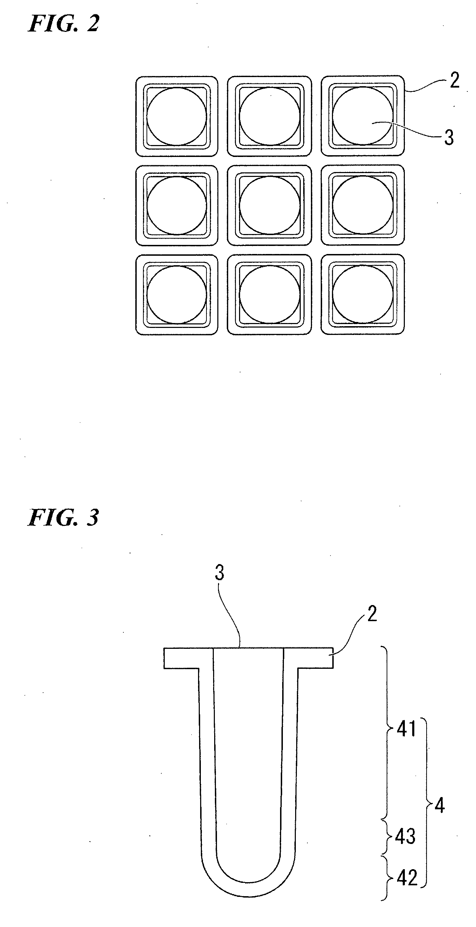

Fig. 1 illustrates a top view of a microwell plate 1 according to the embodiment of the present invention. Fig. 2 illustrates an enlarged view of an II portion of Fig. 1. Fig. 3 illustrates a longitudinal cross-sectional view of a well 2 according to the present embodiment. In the following description, in Fig. 3, an upside of the drawing may be referred to as an upper side, and a downside of the drawing may be referred to as a lower side.

First, the microwell plate 1 according to the present embodiment is described using Fig. 1. The microwell plate 1 is usually used in small-scale cell culture, biochemical experiments, and the like. The microwell plate 1 of the present embodiment is obtained by forming the well 2 on a flat plate that has a thickness equal to or larger than a depth of the well 2. However, the present invention is not limited to this. It is also possible to form the well 2 by recessing a part of a flat plate that has a thickness less than the depth of the well 2.

As illustrated in Figs. 1 and 3, the microwell plate 1 is obtained by forming wells 2 that open on one surface (upper side surface) of a resin-made flat plate. The wells 2 are arranged in n rows and m columns in general. Microwell plates having 6, 12, 24, 48, 96, 384, 1536 or more wells are used. In the following, the microwell plate 1 having 384 wells (16 rows and 24 columns) is described as an example. The microwell plate 1 according to the present embodiment is not limited to this. Microwell plates having various shapes, sizes and numbers of wells may also be use.

The microwell plate 1 of the present embodiment illustrated in Fig. 1 has a flat plate shape with a length of about 125 mm, a width of about 83 mm and a thickness of about 14 mm.

Examples of a material of the microwell plate 1 include polypropylene resin, polyethylene resin, polyolefin-based resin such as ethylene-propylene copolymer or cyclic polyolefin resin, polystyrene, polystyrene-based resin such as acrylonitrile-butadiene-styrene resin, polycarbonate resin, polyethylene terephthalate resin, methacrylic resin such as polymethyl methacrylate resin, vinyl chloride resin, polybutylene terephthalate resin, polyarylate resin, polysulfone resin, polyethersulfone resin, polyether ether ketone resin, polyether imide resin, fluorine-based resin such as polytetrafluoroethylene, polymethylpentene resin, acrylic resin such as polyacrylonitrile, cellulose-based resin such as propionate resin, and the like.

Among these, polystyrene resin is preferable from a viewpoint of moldability and radiation sterilization resistance desired for a culture vessel.

Further, it is preferable to select a transparent resin when performing shape observation of cell aggregates and absorbance measurement and to select a light blocking resin when performing luminescence measurement and fluorescence measurement for each well.

The well 2 is a portion that defines a space 4 for forming a cell aggregate. That is, the well 2 is formed that defines a recess on the upper surface of the microwell plate 1, and the recess becomes the space 4. In the present embodiment, as illustrated in Fig. 3, the space 4 defined in the well 2 has an opening-side portion 41, a bottom-side portion 42 that is formed extending toward an interior of the microwell plate 1, and a transition portion 43 that smoothly connects the opening-side portion 41 and the bottom-side portion 42.

More specifically, the opening-side portion 41 is a portion of which an upper end is at the upper surface of the microwell plate 1 and a lower end is at a place where a polygonal cross section thereof begins to deform toward a circular shape. The above-described place where the cross section begins to deform toward a circular shape is a place where any one of interior angles of the polygonal shape begins to increase.

The bottom-side portion 42 refers to a portion of which an upper end is at a place where an area of the cross section begins to gradually decrease toward an interior and a lower end is the bottom of the well.

The transition portion is a portion extending from the lower end of the opening-side portion 41 to the upper end of the bottom-side portion 42.

As illustrated in Fig. 2, an opening 3 of the well 2 of the present embodiment has a substantially square shape. By making the opening 3 in a polygonal shape other than a circular shape as described above, a capacity of the opening-side portion 41 can be increased. When the opening 3 has a polygonal shape, an effect is achieved that the capacity is increased. Particularly, either a quadrangular, pentagonal or hexagonal shape is preferable because in this case the capacity is increased as compared to a case of a circular shape. Particularly, in the microwell plate 1 of which the number of wells 2 is 384 or more, the effect that the capacity is increased by making the opening 3 in a polygonal shape is large.

In the present embodiment, as illustrated in Fig. 2, the shape of the opening 3 is not formed in a perfect polygonal shape, with each corner being formed rounded. This is preferable because it allows surface treatment and the like to be satisfactorily performed to the well 2.

The opening-side portion 41 of the space 4 has a substantially truncated polyhedral pyramid shape. That is, as illustrated in Fig. 3, a wall surface of a longitudinal cross section of the opening-side portion 41 is inclined toward the bottom side. It is preferable that an inclination angle is as small as possible, for example, about 1 - 2 degrees. By giving some inclination as described above, it will facilitate demolding of the microwell plate 1 from a mold during manufacture. However, it is also possible that the inclination is not provided, for example, when a release property is good by devising a release treatment.

The bottom-side portion 42 of the space 4 is formed in such a manner that the area of the cross section gradually decreases toward the interior. In the present embodiment, the cross section has a circular shape. That is, the bottom-side portion 42 has a so-called U-bottom shape. Such a shape is advantageous to formation of a cell aggregate as it is easy to gather cells at a deepest part. The bottom-side portion 42 is not limited to having a U-shaped bottom. For example, the bottom-side portion 42 may also have a V-shaped bottom. That is, the shape of the longitudinal cross section of the bottom-side portion 42 is not limited to a U shape. For example, even for a V shape, formation of a cell aggregate can be performed.

The transition portion 43 is positioned between the opening-side portion 41 and the bottom-side portion 42. The transition portion 43 is formed to smoothly connect the opening-side portion 41 that has a polygonal cross section and the bottom-side portion 42 that has a circular cross section.

Here, a typical example of dimensions of the well 2 is described.

One side of the opening 3 is about 3.3 mm long. Further, the opening-side portion 41 has a depth of about 8.8 mm, and the bottom-side portion 42 and the transition portion 43 together have a depth of about 3.2 mm. Therefore, a ratio between the depth of the opening-side portion 41 and the combined depth of the bottom-side portion 42 and the transition portion 43 is about 3:1 - 4:1. When the ratio between the depth of the opening-side portion 41 and the combined depth of the bottom-side portion 42 and the transition portion 43 is within the above-described range, in addition to having a bottom portion that allows formation of a cell aggregate, the capacity of the well 2 can be sufficiently ensured.

In the case of the shape of the well 2 of the present embodiment, the capacity of the well 2 is 108.4 µL. On the other hand, for a well of a corresponding size but with a circular opening, the capacity is 87.16 µL. In the formation of a cell aggregate, it is preferable that an amount of a culture medium per well is about 50 µL. However, in the case of the shape of the well of the present embodiment, it is also possible to add a reagent of an amount equal to the culture medium in later evaluation.

Next, a method of using the microwell plate 1 of the present embodiment is described. As an example, in the present embodiment, culturing is performed at 50 µL/well. Therefore, in accordance with the purpose of evaluation that uses cell aggregates, a cell suspension is prepared so that a desired number of cells in 50 µL is ensured.

The so prepared cell suspension is seeded in the microwell plate 1 of 384 wells and culture is started at 37°C and at a CO₂ concentration of 5%. The seeded cells are grown in a non-adherent state, and a cell aggregate is formed at the deepest part of the well 2 in about two days.

Next, screening of drug efficiency evaluation is performed on the microwell plate 1 using the cell aggregate.

As an example, in the present embodiment, CellTiter-Glo (TM) Luminescent Cell Viability Assay (manufactured by Promega Corporation) of an amount same as a culture solution is added to the obtained cell aggregate according to a protocol attached to the product, and an amount of luminescence that is proportional to an intracellular ATP amount of each well is measured. A measured value can be used as an indicator of a number of cells, and a rate of change of the number of cells of each well can be calculated. When a different drug is added to each well, an effect of the drug on cell proliferation can be evaluated.

As described above, according to the microwell plate 1 of the present embodiment, from the formation of a cell aggregate to the screening of drug efficiency evaluation, the processes can be performed without moving the cell aggregate. Therefore, even for screening that uses a large number of specimens, evaluation can be quickly performed.

### Example

Next, embodiments of the present invention are described using an example.

### (Example 1)

Two kinds of, transparent and white, 384-well microwell plates were molded by injection molding using a transparent polystyrene resin (manufactured by PS Japan Corporation, HF77) and using a colored resin that is obtained by mixing 15% of a white pigment, titanium white pigment (manufactured by SUMIKA COLOR Co., Ltd.), with the transparent polystyrene resin.

The obtained microwell plates had a shape with a width of 127.7 mm, a length of 85.5 mm and height of 14.4 mm.

Further, each well had a shape as illustrated in Fig. 3; an opening portion was a quadrangle with each side 3.3 mm long; a depth was 12 mm; a curvature radius of a bottom portion was 1.5 mm; and a well capacity was 108.4 µL.

The depth of the opening-side portion 41 was 8.8 mm; the combined depth of the bottom-side portion 42 and the transition portion 43 was 3.2 mm; and the ratio between the depths was 15:4.

The obtained plates were subjected plasma treatment (oxygen plasma, 10 min) using a plasma treatment device (manufactured by BRANSON/IPC, SERIES7000), and the plate surfaces were imparted with wettability.

Next, in a polypropylene vessel that is light-blocked by a colored resin, BIOSURFINE (R)-AWP (manufactured by Toyo Gosei Co., Ltd.), which is a water-soluble resin, was dissolved in a 25 vol% alcohol aqueous solution, and a water-soluble resin solution of 0.3 wt% was prepared. By using an automatic dispenser (manufactured by Molecular Devices Corporation, AquaMax 2000), the above-described water-soluble resin solution was added to the above-described plates at 80 µL per well, and the plates were immersed for three seconds.

Thereafter, the solution was removed by sucking by using the automatic dispenser and the plates were subjected to primary drying at 25°C for 17 hours.

Next, the water-soluble resin was cured by irradiating the plate with UV light of 250 nm at 1.0 mW/cm²×30 sec using a UV lamp.

Thereafter, the plates were repeatedly washed three times with ultra-pure water and, after being dried, were irradiated with y ray at an absorbed dose of 5.8 kGy (RADIA INDUSTRY Co., Ltd.), and thereby culture vessels (plates) were obtained.

### (Comparative Example)

A culture vessel (plate) was obtained by the same processing steps as Example 1 except that a commercially available microwell plate was used as a molding product. As the commercially available microwell plate, one manufactured by Greiner, 781101, was used.

The plate had a shape with a width of 127.7 mm, a length of 85.5 mm and height of 14.4 mm; and an opening portion of a well was a quadrangle with each side 3.7 mm long. The well had a substantially truncated polyhedral pyramid shape that was formed by the opening-side portion 41 only, with a depth of 11.5 mm and a draft angle of about 1°. Without having the transition portion 43 and the bottom-side portion 42, the well had a bottom surface that is a flat surface of a quadrangle with each side 3.3 mm long.

With respect to the culture vessels obtained in Example 1 and Comparative Example 1, the following evaluation was performed.

### (1) Cell Aggregate Formation Using HepG2 Cells (Human Liver Carcinoma-Derived Cells)

Due to a nutrient requirement of cells during a culture period of cell aggregate culture and an effect of evaporation of a culture medium during the culture, an amount of a culture solution of 50 µL/well or more is required. Therefore, in the following evaluation, the culture was performed with the amount of the culture solution being 50 µL/well.

A cell suspension was prepared that was obtained by dispensing HepG2 cells that were cultured and grown in advance in a culture dish of 90 mmϕ in a culture solution (Dulbecco's modified MEM + 10% fetal bovine serum) at a concentration of 2×10³ cells/mL.

The above-described cell suspension was dispensed at 50 µL/well into all wells of the plate that was molded using the transparent resin, and was cultured at 37°C in a 5% CO₂ atmosphere for three days.

After three days, a state of the cell aggregate in each of the wells was evaluated by microscopic observation.

As a result, in Example 1, a single cell aggregate was observed in all the wells of the plate. On the other hand, in Comparative Example 1, a plurality of cell aggregates were observed in one well.

With respect to the culture vessel that was obtained in Example 1 in which single cell aggregate was obtained, the following evaluation was performed.

### (2) Cell Number Counting By Measuring Luciferase Activity Of Cell Aggregate Using HepG2 Cells

A cell suspension was prepared that was obtained by dispensing HepG2 cells in a culture solution (Dulbecco's modified MEM + 10% fetal bovine serum) at a concentration of 2×10³ cells/mL.

The cell suspension was dispensed at 50 µL/well into the above-described white plate that was molded using the colored resin, and was cultured at 37°C in a 5% CO₂ atmosphere for three days.

After three days, CellTiter-Glo (TM) Luminescent Cell Viability Assay (manufactured by Promega Corporation) of an amount same as the culture solution is added according to a protocol attached to the product, and an amount of luminescence that is proportional to an intracellular ATP amount of each well was measured.

The evaluation was performed for n = 384 and the number of cells was obtained using a calibration curve that was measured in advance.

In Example 1, with respect to an initial number of cells of 1,000, an average number of cells of 4,810, a CV value of 9.5%, and uniform cell proliferation were confirmed.

When a different drug is added to each well, effects of the drugs on cell proliferation can be evaluated. According to the microwell plate of the present embodiment, from the formation of a cell aggregate to the screening of drug efficiency evaluation, the processes can be performed without moving the cell aggregate. Therefore, even for screening that uses a large number of specimens, evaluation can be quickly performed.

### Industrial Applicability

The microwell plate of the present invention is provided with wells that have a proper shape. Therefore, formation of cell aggregates can be satisfactorily performed and a sufficient well capacity can be ensured.

### Description of reference numerals

- 1: microsell plate
- 2: well
- 3: opening
- 4: space
- 41: opening-side portion
- 42: bottom-side portion
- 43: transition portion

## Claims

1. A microwell plate, comprising:
a plate body having a surface on which a plurality of wells are formed, each of the wells having an open end, a bottom end and an interior space extended from the open end to the bottom end,
wherein each of the wells has, in the interior space, an opening-side portion, a bottom-side portion and a transition portion which is continuously connected to the opening-side portion and to the bottom-side portion, the opening-side portion has a polygonal cross section in parallel to the surface of the plate body, and the bottom-side portion has cross sections in parallel to the surface which have areas that gradually decrease toward the bottom end.

2. The microwell plate of Claim 1, wherein the opening-side portion has a first depth, the transition portion has a second depth, the bottom-side portion has a third depth, and each of the wells is formed such that a ratio of the first depth to a sum of the second depth and the third depth is from 3:1 to 4:1.

3. The microwell plate of Claim 1, wherein the polygonal cross section of the opening-side portion is in a shape of one of a quadrangle, a pentagon and a hexagon.

4. The microwell plate of Claim 1, wherein the cross sections of the bottom-side portion have circular shapes.

5. The microwell plate of Claim 1, wherein the plurality of wells comprise 384 or more wells.
